(19) Europäisches Patentamt
European Patent Office
Office européen des brevets

(11) **EP 1 542 053 B1**

(12) **FASCICULE DE BREVET EUROPEEN**

(45) Date de publication et mention
de la délivrance du brevet:
**14.11.2007 Bulletin 2007/46**

(51) Int Cl.:
***G02B 23/24*** *(2006.01)* ***A61B 1/005*** *(2006.01)*

(21) Numéro de dépôt: **04292904.2**

(22) Date de dépôt: **07.12.2004**

(54) **Dispositif de métrologie pour sonde vidéoendoscopique**

Messvorrichtung für eine video-endoskopische Sonde

Measuring device for video-endoscopic probe

(84) Etats contractants désignés:
**DE FR**

(30) Priorité: **11.12.2003 FR 0314526**
**19.12.2003 FR 0315079**
**28.01.2004 FR 0400808**

(43) Date de publication de la demande:
**15.06.2005 Bulletin 2005/24**

(73) Titulaire: **Tokendo**
**13600 La Ciotat (FR)**

(72) Inventeur: **Rovegno, Jean**
**13600 La Ciotat (FR)**

(74) Mandataire: **de Roquemaurel, Bruno**
**Omnipat**
**24, Place des Martyrs**
**De la Résistance**
**13100 Aix en Provence (FR)**

(56) Documents cités:
**US-A- 4 727 859** **US-A- 4 873 572**
**US-A- 5 522 789** **US-A1- 2002 161 284**
**US-B1- 6 361 491**

**Description**

La présente invention concerne les vidéoendoscopes équipés d'un dispositif permettant de mesurer les dimensions d'une cible située dans le champ d'observation du vidéoendoscope.

Elle s'applique notamment, mais non exclusivement à l'endoscopie à vocation industrielle.

On désigne généralement par le terme "endoscope" une sonde souple ou rigide, destinée à être introduite dans une cavité obscure et permettant à son utilisateur d'observer à travers un oculaire l'image d'une cible située dans la cavité. A cet effet, un endoscope intègre un dispositif d'illumination de la cible et un dispositif optique fournissant à l'utilisateur une image de la cible. Le dispositif optique comprend un objectif distal, un dispositif de transport d'images qui peut être rigide constitué d'une série de lentilles, ou souple constitué d'un faisceau de fibres optiques ordonnées, et un oculaire proximal dans lequel l'utilisateur peut observer l'image de la cible. Le dispositif d'illumination comprend généralement un faisceau de fibres d'éclairage dont l'extrémité distale, convenablement orientée à proximité de l'objectif distal, illumine la cible quand son extrémité proximale est connectée à un générateur de lumière.

On désigne par le terme "vidéoendoscope" une sonde souple ou rigide permettant à son utilisateur d'observer sur un écran vidéo l'image d'une cible située dans une cavité obscure. A cet effet, un vidéoendoscope comprend un dispositif d'illumination de la cible identique à celui d'un endoscope et un dispositif d'imagerie fournissant à l'utilisateur une image vidéo de la cible. Le dispositif d'imagerie comprend un dispositif optoélectronique constitué notamment d'un dispositif optique et d'un capteur CCD (Charge Coupled Device) sur la surface sensible duquel se forme l'image de la cible délivrée par ledit dispositif optique, un processeur vidéo relié électriquement au capteur CCD et transformant en un signal vidéo utile le signal électrique délivré par le capteur CCD, un panneau de commande permettant de régler les principaux paramètres de fonctionnement du processeur vidéo, et un moniteur vidéo permettant de visualiser le signal vidéo délivré par le processeur.

On désigne par le terme "sonde vidéoendoscopique" un vidéoendoscope comprenant les éléments suivants :

- un embout distal logeant un dispositif optoélectronique d'imagerie comprenant notamment un objectif et un capteur CCD,
- un tube d'inspection souple, dont l'extrémité distale est solidaire de l'embout distal,
- une poignée de commande solidaire de l'extrémité proximale du tube d'inspection,
- un câble ombilical souple de raccordement dont l'extrémité distale est solidaire de la poignée de commande et dont l'extrémité proximale est destinée à être raccordée à un coffret externe intégrant notamment un générateur de lumière et une source d'alimentation électrique,
- un faisceau de fibres d'éclairage logé dans le câble ombilical, dans la poignée de commande, puis dans le tube d'inspection et dont l'extrémité distale, logée dans l'embout distal, illumine la cible lorsque son extrémité proximale est connectée à un générateur de lumière,
- un processeur vidéo relié électriquement à l'embout distal, et dont la synchronisation est réglée en fonction de la longueur du câble électrique le reliant à l'embout distal,
- un moniteur vidéo relié au processeur vidéo, et
- un panneau de commande permettant de régler le fonctionnement du processeur vidéo et éventuellement du moniteur vidéo.

Les sondes vidéoendoscopiques de conception récente peuvent comprendre en outre les éléments suivants :

- un béquillage distal articulé permettant de modifier l'orientation de l'embout distal de la sonde, la poignée de commande intégrant alors généralement des moyens de commande mécaniques ou électromécaniques permettant d'actionner ce béquillage,
- des têtes optiques interchangeables adaptables sur l'embout distal du tube d'inspection et permettant de modifier le champ optique couvert par le vidéoendoscope et/ou les directions des axes de visée optique et d'illumination du vidéoendoscope, et
- un système numérique de gel, d'enregistrement, de pointage et de traitement d'images susceptible d'être directement géré par le panneau de commande du vidéoendoscope. Un tel système peut en outre être utilisé à des fins de métrologie.

Dans le cadre de l'inspection de pièces mécaniques, il peut être souhaitable de compléter la fonction de visualisation du vidéoendoscope par une fonction de métrologie permettant à l'utilisateur de mesurer directement les dimensions de certains éléments de la pièce mécanique observée. La mise en oeuvre dans un vidéoendoscope d'une fonction métrologie est généralement effectuée à l'aide des moyens suivants :

- un moyen optique qui peut être intégré dans le dispositif optoélectronique distal du vidéoendoscope, et permettant de visualiser un paramètre dont la position dans l'image délivrée par le vidéoendoscope est le reflet de la distance réelle séparant l'extrémité distale de la sonde vidéoendoscopique de la cible que l'on souhaite mesurer,

- un moyen électronique associé au vidéoendoscope et permettant à l'opérateur de pointer sur le moniteur vidéo le paramètre évoqué ci-dessus, ainsi que les extrémités de la cible que l'on souhaite mesurer, et permettant de mettre en oeuvre un algorithme mathématique susceptible de déduire de ces pointages la distance d'observation, puis les dimensions de la cible observée,

Les méthodes de métrologie les plus couramment mises en oeuvre d'abord en endoscopie traditionnelle, puis en vidéoendoscopie sont brièvement évoquées ci-après.

## Mesure directe par déplacement de l'axe de visée

Cette méthode applicable uniquement aux endoscopes rigides à visée latérale consiste à pointer par des moyens optiques (US 4 702 229) ou électroniques (US 4 820 043) les extrémités d'une cible en déplaçant longitudinalement l'endoscope d'une valeur mécaniquement mesurable.

## Mesure par réticulage optique

Cette méthode est également applicable uniquement aux endoscopes rigides et exclusivement aux mesurages répétitifs effectués sur des cibles toujours identiques. Elle consiste à superposer optiquement sur l'image de la cible l'image d'un réticule gradué disposé dans le plan focal distal de l'oculaire de l'endoscope et spécifique de la pièce mécanique formant la cible. Les endoscopes équipés d'un tel réticule sont généralement des endoscopes à visée déviée comportant des dispositifs annexes permettant d'effectuer une mise au point, de faire varier l'angle de visée, de faire tourner l'axe de visée autour de l'axe de l'endoscope et également de faire tourner le réticule. De tels endoscopes ont été décrits dans les brevets US 6 333 812 et FR 2 832 516. Dans le document JP11045349, on a également envisagé de superposer électroniquement sur l'image vidéo de la cible un réseau maillé spécifique de la pièce mécanique à mesurer.

## Mesure par déplacement d'un composant optique

Cette méthode est applicable uniquement aux endoscopes rigides dont l'extrémité proximale du dispositif optique peut éventuellement être solidaire d'un dispositif optoélectronique comprenant notamment un objectif et un capteur CCD. Elle consiste à régler visuellement un paramètre optique en déplaçant longitudinalement l'un des composants optiques du dispositif optique de l'endoscope d'une valeur directement mesurable par des moyens mécaniques, optiques ou électroniques, et à déduire la distance d'observation puis les dimensions de la cible de la valeur de ce déplacement. Le paramètre optique à régler peut être la netteté d'image résultant du déplacement de la lentille de mise au point proximale (US 6 100 972, WO 96/20389) ou de l'objectif distal (US 4 558 691) d'un dispositif optique à faible profondeur de champ. Ce paramètre peut également être le grossissement d'image (US 6 476 979) résultant du déplacement de l'une des lentilles d'un dispositif optique à focale variable.

## Mesure par comparaison

Cette méthode utilisable uniquement en vidéoendoscopie, consiste à déduire les dimensions inconnues d'une cible des dimensions connues d'une cible étalon, et ce à l'aide d'outils électroniques de pointage et de calcul permettant de comparer directement sur l'écran vidéo d'un vidéoendoscope les dimensions des images des deux cibles précitées. Ainsi, dans le brevet US 4 207 594, on utilise pour cible étalon le diamètre du champ objet couvert par l'objectif distal du vidéoendoscope et dans lequel est située la cible à mesurer. D'une manière plus réaliste, les documents GB 2 269 453 et IL 156 074 prévoient d'utiliser en tant que cible étalon une tâche lumineuse de dimensions invariantes obtenue en projetant un rayon lumineux collimaté sur la zone dans laquelle est située la cible à mesurer.

## Mesure par projection sur la cible d'une image auxiliaire

Cette méthode, utilisée à l'origine en endoscopie traditionnelle est par exemple décrite dans les brevets DE 2 847 561 et US 4 660 982. Elle consiste à projeter sur la cible visée par l'endoscope une image non collimatée générée par un masque associé à un objectif intégré dans l'extrémité distale du dispositif d'illumination de l'endoscope. La distance d'observation et les dimensions de la cible peuvent alors être déduites du positionnement et des dimensions, dans le champ image de l'oculaire de l'endoscope, d'une part de l'image de la cible et d'autre part de l'image de l'image auxiliaire. Cette méthode a été appliquée en vidéoendoscopie suivant des modalités de mise en oeuvre décrites dans les brevets US 4 980 763 et US 5 070 401. Parallèlement, la mesure par une méthode analogue d'une cible située dans le champ d'observation d'une caméra vidéo associée à un projecteur laser appliquant sur la cible une tâche lumineuse non collimatée a été décrite dans le brevet FR 2 630 538. Dans ces deux méthodes, l'utilisation d'outils électroniques de calcul et de pointage sur écran vidéo des images de la cible et de l'image auxiliaire a permis de simplifier la mise en oeuvre des procédures de mesure. Le brevet DE 3629435 décrit une autre méthode de mesure utilisable en endoscopie traditionnelle comme en vidéoendoscopie, qui combine une méthode mesure par comparaison (utilisation d'une cible étalon formée par les images de deux rayons laser collimatés et parallèles à l'axe optique) et une méthode de mesure par projection d'une image auxiliaire (image d'un troisième rayon laser collimaté et incliné par rapport à l'axe optique).

Mesure stéréo traditionnelle à deux voies optiques

Cette méthode consiste à former sur la surface sensible du capteur CCD distal d'une sonde vidéoendoscopique deux images de la cible observée sous des angles différents grâce à deux voies optiques distales distinctes. La distance d'observation et les dimensions de la cible peuvent alors être déduites à l'aide d'outils électroniques de pointage et de calcul des positions relatives et des dimensions sur l'écran vidéo de la sonde de ces deux images. Ces deux images peuvent être simultanément générées par deux objectifs distincts disposés dans l'extrémité distale de la sonde (US 4 873 572, US 2002/0137986, US 6 063 023, US 6 361 491), ou de façon séquentielle grâce à la mise en oeuvre alternée de deux pupilles intégrées dans l'objectif distal de la sonde et disposées symétriquement par rapport à l'axe optique (US 5 222 477).

Mesure par dédoublement d'image

Il existe également des procédés de visualisation stéréo mis en oeuvre non pas en endoscopie, mais dans le domaine de la télévision en relief. Ces procédés consistaient à l'origine (par exemple d'après les brevets US 3 932 699, FR 2 619 664, FR 2 704 951, FR 2 705 006) à associer deux réseaux lenticulaires homothétiques comprenant le même nombre d'éléments optiques unitaires de dédoublement d'image, le plus petit de ces réseaux étant placé devant l'objectif d'une caméra vidéo de prise de vues, tandis que le plus grand était collé sur le tube d'un récepteur de télévision associé à ladite caméra. Une méthode de mesure, dérivée de ces procédés de visualisation a été décrite dans le brevet US 4 993 830. La mise en oeuvre simultanée de moniteurs vidéo de type LCD constitués d'une matrice de pixels et de programmes de traitement d'image permettant de gérer le signal reçu par chacun de ces pixels a permis d'améliorer ces procédés d'une part en associant finement la structure du réseau lenticulaire de réception à celle de la matrice de pixels du moniteur vidéo, et d'autre part en n'utilisant plus qu'un seul élément optique de dédoublement d'image au niveau de la caméra vidéo de prises de vues et donc d'améliorer la qualité optique dudit élément. C'est ainsi que l'inventeur français Pierre ALLIO, titulaire d'un grand nombre de brevets dans ce domaine, a présenté dans le numéro d'Avril 2003 de la revue française "Science & vie" un dispositif optique de dédoublement d'image destiné à être placé devant l'objectif d'une caméra de télévision et comprenant:

- un élément optique proximal présentant une face distale plane et une face proximale dotée d'un profil transversal concave,
- un élément optique distal présentant une face distale dotée d'un profil transversal convexe disposé perpendiculairement par rapport au profil concave de l'élément proximal et une face proximale dans laquelle sont usinées deux bandes transversales parallèles et adjacentes présentant chacune un profil concave identique et disposées perpendiculairement par rapport au profil concave de l'élément proximal.

Déjà évoquée dans le brevet DE 3 432 583, la mise en oeuvre d'un procédé de mesure utilisant un dispositif discret de dédoublement d'image constitué d'un simple composant optique présentant une surface distale plane et une face proximale en forme de delta à arête saillante a été décrite dans les brevets US 6 411 327 et US 2002/0089583. Conçu non pas pour être intégré dans une sonde vidéoendoscopique mais pour être placé devant l'objectif d'une caméra vidéo, ce dispositif est destiné à mesurer la distance d'observation d'une cible située à proximité de l'axe optique d'une caméra vidéo traditionnelle.

Il s'avère que les dispositifs stéréo permettant de former simultanément sur le capteur CCD deux images d'une cible sous deux angles de visée différents offrent de meilleures précision et répétitivité des mesures. Toutefois, les trois dispositifs stéréo décrits ci-dessus et permettant d'aboutir à un tel résultat présentent des inconvénients gênants, voire rédhibitoires en ce qui concerne leur intégration dans une sonde vidéoendoscopique de très faible diamètre. En effet, l'intégration de deux objectifs à visée axiale dans l'extrémité distale d'une sonde vidéoendoscopique nécessite la mise en oeuvre de lentilles de très faible diamètre et ne permet pas d'utiliser la totalité de la surface sensible du capteur CCD associé aux objectifs. Ces limitations ne vont pas dans le sens d'une optimisation de la précision du processus de mesure. Par ailleurs, l'intégration dans l'extrémité distale d'une sonde vidéoendoscopique d'un dispositif de dédoublement d'image présentant des profils rectilignes transversaux concaves s'avère délicate à réaliser en raison des difficultés de miniaturisation d'un tel dispositif. Par contre, la mise en oeuvre d'un dispositif discret de dédoublement d'images constitué d'un prisme présentant une section en forme de delta ne pose aucun problème de miniaturisation. Cependant, le champ optique de mesurage d'un tel prisme s'avère, par principe, inférieur à 15°, valeur nettement insuffisante pour un système de mesure endoscopique.

La souplesse d'utilisation d'une sonde vidéoendoscopique suppose la possibilité d'adapter sur son extrémité distale des têtes d'observation amovibles et interchangeables couvrant différentes valeurs de champ et/ou d'angle de visée. Il est évidemment souhaitable que le dispositif optique nécessaire à la mise en oeuvre d'une procédure de mesure puisse, lui aussi, être intégré dans une tête amovible, sachant que dans le cas des dispositifs mécaniques permettant de positionner et de verrouiller les têtes amovibles par rapport au capteur CCD de la sonde doivent répondre à des exigences de précision plus sévères. D'une manière générale, les dispositifs de verrouillage d'une tête amovible sur l'extrémité distale d'une sonde vidéoendoscopique doivent satisfaire aux contraintes suivantes :

- mise en continuité des voies optiques et des voies d'illumination de la sonde vidéoendoscopique et de la tête amovible, fonction nécessitant la mise en oeuvre simultanée d'un verrouillage longitudinal et d'une indexation latérale,
- prévention de toute possibilité de déverrouillage accidentel de la tête amovible,
- absence de pollution du capteur CCD par des rayons lumineux parasites émanant de la voie d'illumination de la sonde.

Les moyens mécaniques mis en oeuvre pour répondre à ces contraintes varient en fonction de la structure optique du couple sonde / tête amovible. Le plus souvent, comme c'est le cas dans le brevet US 4 727 859, le capteur CCD de la sonde est fixement associé à un dispositif optique distal présentant un encombrement frontal inférieur à celui du capteur CCD. Ce dispositif optique peut dans ces conditions être logé dans la partie distale de la sonde qui peut alors présenter un diamètre inférieur à celui de la sonde afin de pouvoir être coiffé par l'extrémité tubulaire proximale des têtes amovibles. Cette architecture présente l'avantage de simplifier les dispositifs de verrouillage et l'inconvénient de devoir loger dans les têtes amovibles des dispositifs optiques complémentaires à celui intégré à demeure dans la sonde. Sur le plan purement optique, il s'avère techniquement plus avantageux de loger l'ensemble du système optique dans les têtes amovibles qui viennent alors se fixer directement sur le capteur CCD intégré à la sonde, sachant que les dispositifs mécaniques de verrouillage seront alors plus délicats à réaliser.

La présente invention vise à réaliser un dispositif optique de dédoublement d'image intégrable dans l'extrémité distale d'une sonde vidéoendoscopique de faible diamètre, présentant les avantages suivants:

- offrir un champ de mesurage significatif,
- permettre l'utilisation pour les mesures d'une portion significative de la surface sensible du capteur CCD,
- être intégrable dans des têtes optiques interchangeables à visée axiale ou à visée déviée susceptibles d'être connectées sur l'extrémité distale d'une sonde vidéoendoscopique, et comportant un nombre minimum de composants optiques de manière à présenter un encombrement restreint.

Cet objectif est atteint par la prévision d'un objectif distal de vidéoendoscope pour former sur la surface sensible d'un capteur vidéo logé dans l'embout distal d'un vidéoendoscope une unique image composite formée de deux images d'une cible observée, vue sous deux angles différents.

Selon l'invention, l'objectif comprend un unique composant optique de dédoublement d'image et une lentille convergente proximale disposée en position proximale par rapport au composant optique de dédoublement, le composant optique de dédoublement d'image étant constitué de deux portions de lentille convergente identiques incluant chacune l'axe optique de la lentille convergente d'où elles sont issues, et d'un élément central opaque maintenant les deux portions de lentilles espacées l'une de l'autre, de manière à ce que les axes optiques des deux portions de lentilles soient espacés l'un de l'autre, la lentille convergente proximale coopérant avec chacune des deux portions de lentilles pour former l'image composite.

Selon un mode préféré de réalisation de l'invention, le composant optique de dédoublement d'image est associé à un masque opaque présentant deux orifices formant deux diaphragmes, centrés respectivement sur les axes optiques des deux portions de lentilles

Selon un mode préféré de réalisation de l'invention, les dimensions de l'élément central opaque sont déterminées de manière à éviter un chevauchement de champs images produits par les portions de lentilles au travers de la lentille convergente proximale.

Selon un mode préféré de réalisation de l'invention, le composant optique de dédoublement d'image est obtenu par une découpe suivant un cylindre de section droite circulaire, centré sur l'élément opaque et dont l'axe longitudinal est parallèle aux axes optiques des portions de lentilles.

Avantageusement, le dispositif de dédoublement d'image comprend en outre une lentille divergente disposée en position distale par rapport au composant optique de dédoublement.

Selon un mode préféré de réalisation de l'invention, le dispositif de dédoublement d'image comprend en outre des moyens de réflexion disposés en position distale, pour introduire une déviation de l'angle de visée du dispositif de dédoublement d'image.

Avantageusement, le dispositif de dédoublement d'image comprend en outre une lentille divergente disposée en position distale par rapport aux moyens de réflexion.

Selon un mode préféré de réalisation de l'invention, le dispositif de dédoublement d'image se présente sous la forme d'une tête optique comprenant des moyens de fixation pour se fixer de manière amovible sur l'embout distal d'un vidéoendoscope, pour former une image dédoublée sur la surface sensible d'un capteur vidéo logé dans l'embout distal.

Avantageusement, les moyens de fixation sont du type doigt destiné à coopérer avec une fente en forme de baïonnette et comprennent des moyens d'accouplement pour assurer un alignement correct de l'axe optique de la tête optique avec le capteur vidéo.

Selon un mode préféré de réalisation de l'invention, le dispositif de dédoublement d'image comprend un faisceau de fibres d'éclairage dont l'extrémité distale est destinée à éclairer la cible et dont l'extrémité proximale est destinée à être couplée à l'extrémité distale d'un faisceau de fibres d'éclairage équipant le vidéoendoscope, les moyens d'accouplement assurant un alignement correct du faisceau de fibres d'éclairage avec celui du vidéoendoscope.

Avantageusement, les moyens de fixation sont formés sur un tube de verrouillage mobile axialement sur la tête optique entre des positions distale et proximale, et ramené vers la position distale par des moyens de rappel élastiques, les moyens d'accouplement étant de type mâle-femelle et étant disposés de manière à arriver en position accouplée en fin de course de verrouillage des moyens de verrouillage, sous l'effet des moyens de rappel élastiques.

L'invention concerne également un vidéoendoscope comprenant un embout distal logeant un capteur vidéo. Selon l'invention, le capteur vidéo est couplé à un objectif tel que défini ci-avant.

Selon un mode préféré de réalisation de l'invention, le vidéoendoscope comprend des moyens de calcul de la distance d'observation d'un point de la cible à partir de la distance sur un écran de visualisation sur lequel est affichée l'image composite de la cible formée sur le capteur vidéo entre deux points images du point de la cible, générés par le dispositif de dédoublement d'image.

Selon un mode préféré de réalisation de l'invention, le vidéoendoscope comprend des moyens de calcul de la distance entre un premier et un second points de la cible à partir des positions respectives sur un écran de visualisation sur lequel est affichée l'image composite de la cible formée sur le capteur vidéo de deux points images du premier point de la cible et de deux points images du second point, générés par le dispositif de dédoublement d'image.

Selon un mode préféré de réalisation de l'invention, le vidéoendoscope comprend des moyens pour appliquer à une portion de l'image composite représentant la cible observée, un traitement d'agrandissement, de manière à afficher une seule des deux images de la cible, constituant l'image composite.

Selon un mode préféré de réalisation de l'invention, le vidéoendoscope comprend une paire de lunettes de vision stéréo comprenant deux mini écrans vidéo vers lesquels sont envoyées respectivement les deux images dédoublées de la cible, après avoir subi un traitement d'agrandissement.

Avantageusement, le capteur vidéo est un capteur CCD.

Un mode de réalisation préféré de l'invention sera décrit ci-après, à titre d'exemple non limitatif, avec référence aux dessins annexés dans lesquels :

La figure 1 représente en vue en perspective éclatée, une partie d'un composant optique de dédoublement d'image selon l'invention ;

Les figures 2a et 2b montrent respectivement en coupe longitudinale et en vue de face, la structure du composant optique selon l'invention, intégrant la partie représentée sur la figure 1 ;

Les figures 3a et 3b montrent respectivement en coupe longitudinale et en vue de face, la structure d'une variante du composant optique représenté sur les figures 2a et 2b ;

La figure 4 représente la distribution des champs optiques véhiculés par le composant optique représenté sur les figures 3a et 3b;

La figure 5 illustre la relation géométrique à la base de l'algorithme de calcul de la distance entre un point situé dans le champ optique de mesure et l'extrémité distale du composant optique représenté sur les figures 3a et 3b ;

La figure 6 représente schématiquement un capteur CCD distal muni d'un objectif à dédoublement d'image selon l'invention, visant deux objets alignés sur l'axe optique de l'objectif;

La figure 7 représente un écran vidéo sur lequel est formée une image dédoublée générée par le capteur CCD dans les conditions illustrées par la figure 6 ;

La figure 8 montre l'image représentée sur la figure 7, agrandie d'un facteur 2 ;

La figure 9 représente schématiquement une paire de lunettes de vision stéréo ;

La figure 10 représente schématiquement en coupe longitudinale la structure de la partie distale d'une sonde vidéoendoscopique susceptible d'être équipée de têtes distales interchangeables ;

Les figures 11 et 12 représentent en coupe longitudinale la structure d'une tête distale de mesure à visée axiale et latérale, respectivement, intégrant le composant optique de dédoublement d'image montré sur les figures 3a et 3b ;

Les figures 13 et 14 montrent en coupe longitudinale des variantes des têtes distales de mesure représentées respectivement sur les figures 11 et 12 ;

La figure 15 représente plus en détail en perspective la structure de l'embout distal de sonde endoscopique montré sur la figure 10 ;

Les figures 16 et 17 représentent plus en détail en perspective la structure des têtes distales de mesure à visée axiale et latérale, montrées respectivement par les figures 11 et 12 ou 13 et 14 ;

La figure 18 montre en coupe longitudinale partielle la structure de l'embout distal de sonde endoscopique montré sur la figure 15 ;

La figure 19 montre en coupe longitudinale la structure de la tête distale de mesure à visée axiale représentée sur la figure 16.

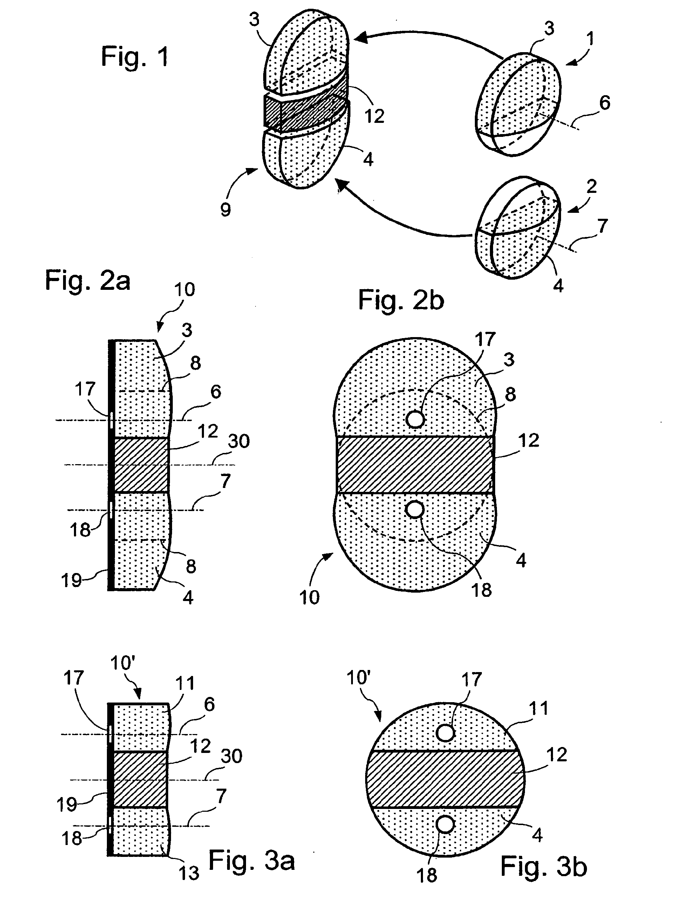

La figure 1 représente schématiquement la structure d'un composant optique à dédoublement d'image 10 selon l'invention.

Le composant optique 9 montré sur cette figure est obtenu en solidarisant un élément central opaque 12 avec deux portions latérales identiques 3, 4, de lentilles 1, 2, supérieures à une demi-lentille ou demi lune, de manière à inclure l'axe optique 6 ,7 des lentilles 1, 2. Chacune de ces portions de lentilles est obtenue en découpant une lentille convergente 1, 2 par exemple suivant un plan de coupe parallèle à l'axe optique de la lentille, la lentille convergente présentant une face plane et une face convexe. Le composant optique 9 montré sur la figure 1 présente ainsi deux plans de symétrie perpendiculaires, à savoir un plan médiateur de l'élément central 13 coupant chacune des deux portions de lentilles et l'élément central en deux parties symétriques et un plan médiateur de l'élément central de part et d'autre duquel les deux portions de lentilles sont disposées symétriquement.

Les figures 2a et 2b montrent en vues de face et de profil un composant optique 10 obtenu en associant le composant optique 9 représenté sur la figure 1 à un masque opaque 19 collé sur la face plane du composant 9, ce masque présentant deux orifices circulaires 17, 18 servant de diaphragmes d'ouverture qui sont centrés sur les axes optiques 6, 7 des deux portions de lentilles 3, 4.

Les figures 3a et 3b représentent une variante 10' du composant optique de dédoublement d'image selon l'invention. Le composant optique montré sur cette figure est obtenu en découpant le composant optique 10 représenté sur les figures 2a et 2b suivant un cylindre 8 de section droite circulaire, centré sur ce dernier et dont l'axe longitudinal est parallèle aux axes optiques des portions de lentilles 3, 4. On obtient ainsi un composant ayant les dimensions d'une lentille.

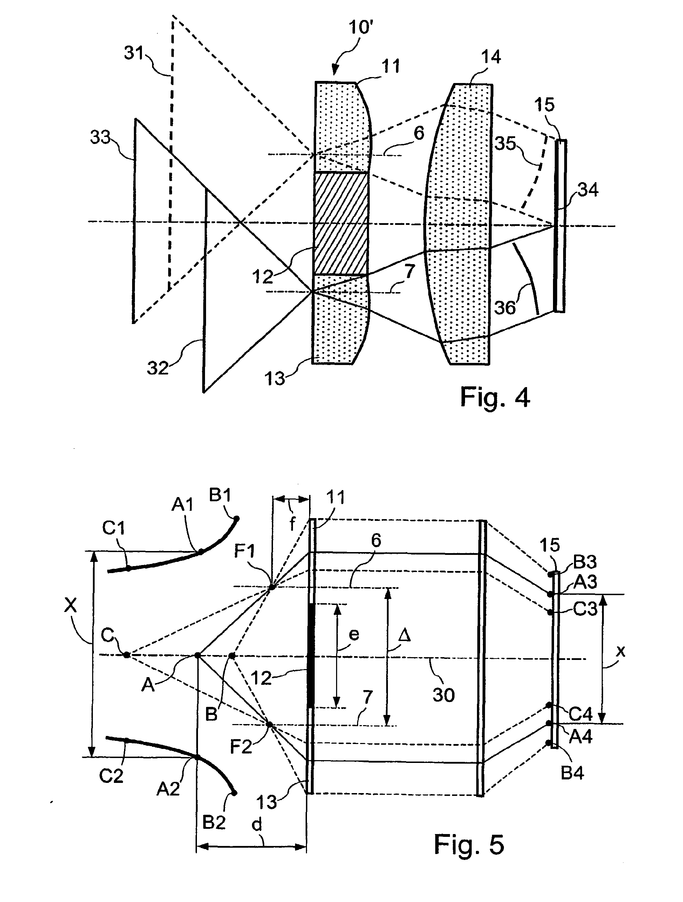

La figure 4 illustre schématiquement la distribution des champs optiques véhiculés par le dispositif optoélectronique de mesure selon l'invention. Ce dispositif comprend un capteur CCD proximal 15, une lentille convergente centrale 14 et le composant optique distal 10 ou 10' à dédoublement d'image décrit en référence aux figures 2a, 2b ou 3a, 3b. Dans le système optique regroupant la portion de lentille latérale 11 du composant optique distal 10, 10' et la lentille centrale 14, le champ objet 31 couvert par la portion de lentille 11 forme sur le capteur CCD proximal 15 un champ image 35 qui couvre sensiblement la moitié de la surface sensible 34 du capteur CCD. De la même façon, dans le système optique regroupant la portion de lentille latérale 13 du composant optique distal 10, 10' et la lentille centrale 14, le champ objet 32 couvert par la portion de lentille 12 forme sur la surface sensible du capteur CCD un champ image 36 qui couvre sensiblement l'autre moitié de la surface sensible 34 du capteur CCD non couverte par le champ image 35. Il en résulte que tout point objet situé dans le champ objet 33 situé dans l'intersection des deux champs objet 31, 32 génère deux points image distincts sur la surface sensible du capteur CCD 15.

Il convient de remarquer que la répartition des champs images 35, 36 dans deux moitiés distinctes de la surface sensible du capteur CCD 15 (et donc l'absence de chevauchement de ces deux images au centre de la surface sensible du capteur) est obtenue en calculant correctement d'une part l'épaisseur et la hauteur de l'élément central opaque 12, et d'autre part, l'écart séparant les axes optiques 6 et 7 des deux portions de lentilles latérales 11 et 13

Il est intéressant de noter que la structure du dispositif optoélectronique distal de mesure axiale décrit ci-avant découle directement de celle d'un dispositif optoélectronique distal de visualisation axiale, intégré dans l'extrémité distale d'une sonde vidéoendoscopique traditionnelle. En effet, un tel dispositif de visualisation comprend un capteur CCD proximal 15 et un objectif distal constitué au moins d'une lentille convergente proximale 14 et d'une lentille convergente distale. La transformation du dispositif de visualisation en un dispositif de mesure est donc effectuée simplement en remplaçant la lentille convergente distale par le composant optique 10, 10' décrit en référence aux figures 2a, 2b ou 3a, 3b. Dans ces conditions, et toujours par analogie avec les dispositifs optoélectroniques distaux de visualisation axiale utilisés en vidéoendoscopie traditionnelle, il convient de signaler que le dispositif optoélectronique distal de mesure axiale selon la présente invention peut aisément être amélioré par l'adjonction d'une lentille divergente distale qui aura pour effet d'élargir les champs optiques 31, 32 et donc également le champ optique de mesure commun 33.

La figure 5 illustre schématiquement le processus géométrique mis en oeuvre dans le dispositif optoélectronique distal de mesure axiale selon la présente invention. Dans ce processus, un point objet A situé sur l'axe optique 30 du champ de mesure 33 forme un couple de points image A3, A4 sur la surface sensible du capteur CCD 15. Compte tenu du décalage par rapport à l'axe optique 30 du point focal F1 de la portion de lentille distale 11, cette portion de lentille génère une image virtuelle A1 du point objet A, image virtuelle dont le décalage par rapport à l'axe optique 30 est fonction de la distance d séparant le point objet A de l'extrémité distale du dispositif optoélectronique de mesure. La reprise de l'image virtuelle A1 par la lentille centrale 14 génère un point image A3 sur la surface sensible du capteur CCD 15. De la même façon, et compte tenu du décalage par rapport à l'axe optique 30 du point focal F2 de la portion de lentille distale 13, cette portion de lentille génère une image virtuelle A2 du point objet A. La reprise de l'image virtuelle A2 par la lentille centrale 14 génère un point image A4 sur la surface sensible du capteur CCD 15. De la même façon, un point objet B situé à une distance d'observation plus courte que le point A, est associé à deux images virtuelles B1, B2, puis à deux points image B3, B4 sur la surface sensible du capteur CCD 15. Un point objet C, situé à une distance d'observation plus grande que celle du point A est associé à deux images virtuelles C1, C2, puis à deux points image C3, C4.

Il convient de noter, bien que cela n'ait pas été mis en évidence sur la figure 4, qu'un point objet décalé par rapport à l'axe optique 30 et situé dans le champ de mesure 33 à l'aplomb du point objet A génère également deux images virtuelles dont l'écart sera identique à celui séparant les deux images virtuelles A1, A2, et forme sur la surface sensible du capteur CCD 15 deux points image dont l'écart est identique à celui séparant les deux points image A3, A4.

On peut également remarquer que les images virtuelles A1, B1, C1 d'une part et d'autre part A2, B2, C2 se répartissent sur deux courbes de forme hyperbolique, symétriques par rapport à l'axe optique 30. Cette répartition prouve que la précision de la mesure de la distance d'observation d'un point objet situé dans le champ de mesure 33, mesure découlant in fine de la mesure de l'écart séparant les deux images virtuelles dudit point objet, sera plus précise pour de courtes distances d'observation (tout point objet situé entre A et B par exemple) que pour des distances d'observation plus longues (tout point objet situé par exemple entre A et C). Dans ces conditions, il est prudent de prévoir dans la procédure de calcul de la distance d'observation une alarme qui se déclenche dès que l'écart séparant les deux points image d'un même point objet, affichés sur l'écran vidéo de la sonde vidéoendoscopique est inférieur à une valeur de seuil prédéterminée correspondant à une dégradation inacceptable de la précision de mesure de la distance d'observation.

La figure 5 illustre également la relation géométrique dont découle l'algorithme permettant de calculer la distance d'observation d séparant un point objet A situé dans le champ optique de mesure 33, de l'extrémité distale du dispositif optoélectronique distal de mesure axiale selon la présente invention. Cette distance d'observation est basée sur la connaissance de la distance focale f des portions de lentilles 11, 13, de la connaissance de l'écart $\Delta$ séparant les deux axes optiques 6, 7 des portions de lentilles 11, 13, de la mesure de la distance X séparant les deux images virtuelles A1, A2. La distance d'observation d est obtenue à l'aide de l'équation suivante:

$$d = f X /(X - \Delta) \qquad\qquad (1)$$

En pratique, la distance X est obtenue en mesurant l'écart effectif séparant les deux points image affichés à l'écran vidéo de la sonde vidéoendoscopique, cet écart étant proportionnel à l'écart x séparant les deux points image A3, A4 formés sur la surface sensible du capteur CCD 15. L'écart x est lui-même proportionnel à l'écart X séparant les deux images virtuelles A1, A2 du point objet A.

La mesure de la longueur réelle d'un défaut (tel que par exemple une crique), type de mesure qui constitue la vocation première des sondes vidéoendoscopiques selon la présente invention, met en oeuvre des procédures de calcul et d'étalonnage qui ne seront pas décrites dans la présente description. Pratiquement, une telle mesure de longueur nécessite en effet un calcul préalable des coordonnées spatiales réelles des extrémités du défaut. Outre les distances d'observation desdites extrémités (distances dont le principe de calcul a été décrit ci-avant), un tel calcul exige la prise en compte d'autres paramètres qui peuvent être obtenus soit à partir de la structure même de la sonde vidéoendoscopique (grandissement global du système regroupant le dispositif optoélectronique distal de mesure, le processeur vidéo, et l'écran vidéo de la sonde), soit à l'aide de pointages sur l'écran vidéo des extrémités de l'image du défaut (angles de visée des extrémités du défaut), soit à l'aide d'un étalonnage préalable (correction des distorsions optiques).

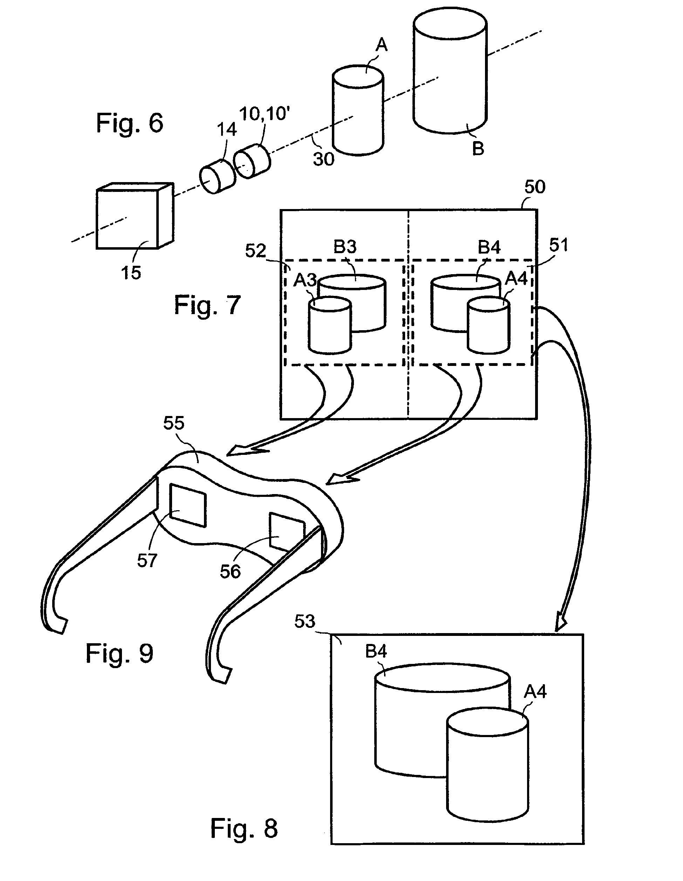

La figure 6 représente un dispositif optoélectronique distal d'un vidéoendoscope comprenant le capteur CCD 15 et un objectif de dédoublement d'image comportant au moins la lentille convergente 14 et le composant optique 10, 10' de dédoublement d'image, visant une cible constituée dans l'exemple de la figure par deux cylindres A et B alignés sur l'axe optique 30.

La figure 7 représente l'écran vidéo 50 affichant l'image composite générée par le capteur 15. Cette image composite montre dans deux images distinctes 51, 52, la cible dédoublée A3, B3 et A4, B4, observée sous deux angles différents par le composant 10, 10'. Un tel écran est utilisé pour effectuer sur l'image dédoublée les différents pointages nécessaires aux procédés de calcul de métrologie, permettant de déterminer par exemple la distance d'observation des cylindres A et B ou leurs diamètres respectifs.

Toutefois, la structure composite d'une telle image composite se prête mal à une simple observation de la cible. Pour résoudre ce problème tout en évitant d'avoir à remplacer la tête optique intégrant l'objectif de dédoublement d'image 10 ou 10', et 14, par une tête optique sans un tel objectif, l'invention propose d'effectuer au cours du traitement du signal vidéo pour générer l'image composite, un traitement d'agrandissement de rapport 2 appliqué à une portion d'image 51 représentant la cible observée, occupant un quart de la surface de visualisation de l'écran vidéo. L'image 53 ainsi obtenue et affichée est représentée sur la figure 8. De cette manière, on affiche une seule des deux images de la cible, constituant l'image dédoublée, par exemple celle de droite montrant la partie A4, B4 de la cible dédoublée.

Les images 51, 52 dédoublées de la cible qui occupent sensiblement un quart de la surface de visualisation de l'écran vidéo, peuvent également subir simultanément un traitement d'agrandissement de rapport 2, afin d'être visualisées par deux mini écrans vidéo 56, 57 intégrés dans une paire de lunettes 55 de vision stéréo montrée sur la figure 9.

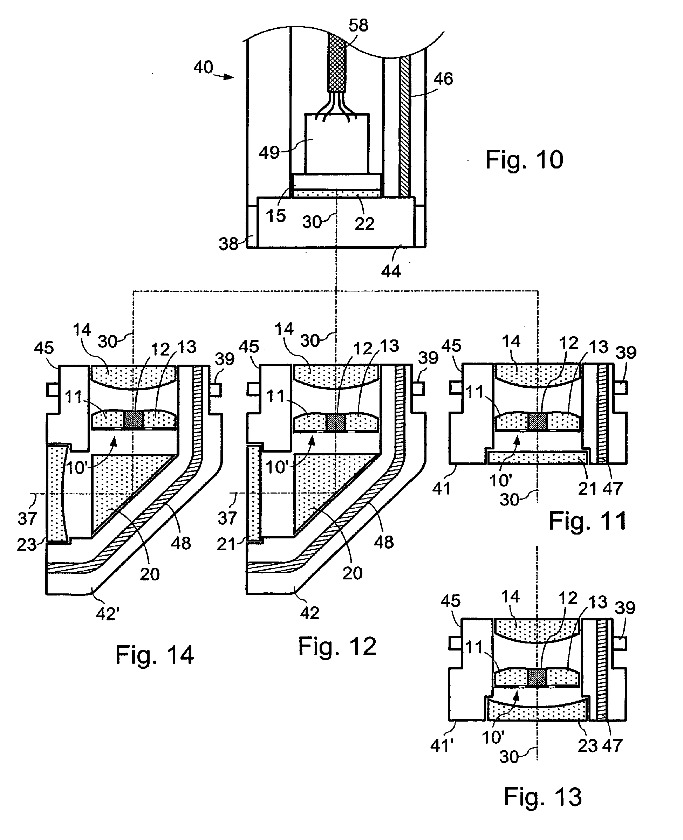

La figure 10 illustre schématiquement la structure de la partie distale 40 d'une sonde vidéoendoscopique susceptible d'être équipée de têtes distales interchangeables. La partie distale 40 loge d'une part l'extrémité distale 46 du faisceau de fibres d'éclairage intégré dans ladite sonde et d'autre part un capteur CCD 15 protégé par une paroi transparente distale 22 et électriquement relié au processeur vidéo de la sonde par l'intermédiaire d'un micro circuit électronique d'interface 49 et d'un câble multiconducteurs 58. La partie distale 40 est en outre équipée d'un dispositif mécanique distal d'indexation et de verrouillage permettant l'adaptation de têtes distales interchangeables.

Dans la configuration illustrée dans la figure 10, le dispositif mécanique distal d'indexation et de verrouillage comprend un tube cylindrique distal 44 présentant deux fentes longitudinales ouvertes 38 en forme de baïonnette et diamétralement opposées. Le tube 44 loge l'extrémité proximale cylindrique des têtes distales interchangeables, cette extrémité disposant de deux tétons latéraux 39 diamétralement opposés (figures 11 et 12), venant s'engager, puis se verrouiller dans les deux fentes longitudinales 38 en forme de baïonnette. Les positionnements relatifs desdites fentes et desdits tétons étant calculés de façon à assurer en position de verrouillage la continuité des voies d'illumination et des voies optoélectroniques de la sonde vidéoendoscopique et des têtes distales interchangeables.

La partie distale 40 de la sonde vidéoendoscopique illustrée à titre d'exemple dans la figure 10 est dépourvue de tout dispositif optique, l'ensemble des composants optiques associés au capteur CCD 15 étant dans ces conditions systématiquement intégré dans les têtes distales interchangeables. On peut imaginer un autre mode d'organisation dans lequel le capteur CCD 15 de la sonde vidéoendoscopique serait systématiquement associé à un composant optique distal, organisation qui allégerait d'autant la structure optique des têtes distales interchangeables.

La figure 11 illustre schématiquement la structure d'une tête distale de mesure axiale 41 intégrant la lentille proximale convergente 14 en position proximale, le composant optique 10 ou 10' de dédoublement d'image décrit en référence aux figures 2a, 2b ou 3a, 3b, et une paroi transparente distale de protection 21. Il est à noter que la paroi distale 21 qui pourrait être remplacée par une lentille divergente assurant l'élargissement du champ optique de mesure couvert par la tête distale 41' (voir figure 13).

La partie cylindrique proximale 45 de la tête distale 41, est destinée à venir se loger dans le tube cylindrique 44 constituant l'extrémité distale de la sonde vidéoendoscopique. Elle dispose à cet effet de deux tétons cylindriques latéraux externes 39 diamétralement opposés, destinés à venir se loger dans les deux fentes 38 diamétralement opposées en forme de baïonnette ménagées dans le tube cylindrique distal 44. Le système de verrouillage ainsi réalisé assure d'une part la mise en continuité du faisceau de fibres d'illumination axiale 47 logé dans la tête distale 41 avec le faisceau de fibres d'éclairage logé dans la sonde vidéoendoscopique, et d'autre part l'indexation correcte du composant optique de dédoublement d'image 10, 10' par rapport au capteur CCD 15 distal de la sonde vidéoendoscopique.

La figure 12 illustre schématiquement la structure d'une tête distale de mesure latérale 42. Cette tête intègre un système optique comprenant successivement d'une lentille proximale convergente 14, le composant optique de dédoublement d'image 10 ou 10' décrit en référence aux figures 2a, 2b ou 3a, 3b, un prisme déviateur 20 et une paroi transparente distale de protection 21.

Comme précédemment, la paroi distale 21 peut être remplacée par une lentille divergente 23 assurant l'élargissement du champ optique de mesure couvert par la tête distale 42' (voir figure 13).

Le système de verrouillage de la tête distale 42 sur la sonde vidéoendoscopique à laquelle elle est associée, est identique à celui décrit ci-avant en référence à la figure 6, de manière à assurer également la mise en continuité du faisceau de fibres d'illumination latérale 48 logé dans la tête distale 42 avec le faisceau de fibres d'éclairage 46 logé dans la sonde vidéoendoscopique, et l'indexation correcte du composant optique de dédoublement d'image 10, 10' par rapport au capteur CCD 15 distal de la sonde vidéoendoscopique.

Il convient de remarquer que le dispositif de dédoublement d'image 10, 10' selon l'invention assure en même temps la fonction de l'une des deux lentilles de l'objectif du vidéoendoscope. Il en résulte que les têtes optiques de métrologie 41, 42, 41' et 42' selon l'invention sont plus compactes que celles de l'art antérieur intégrant un composant optique n'assurant que la fonction de métrologie.

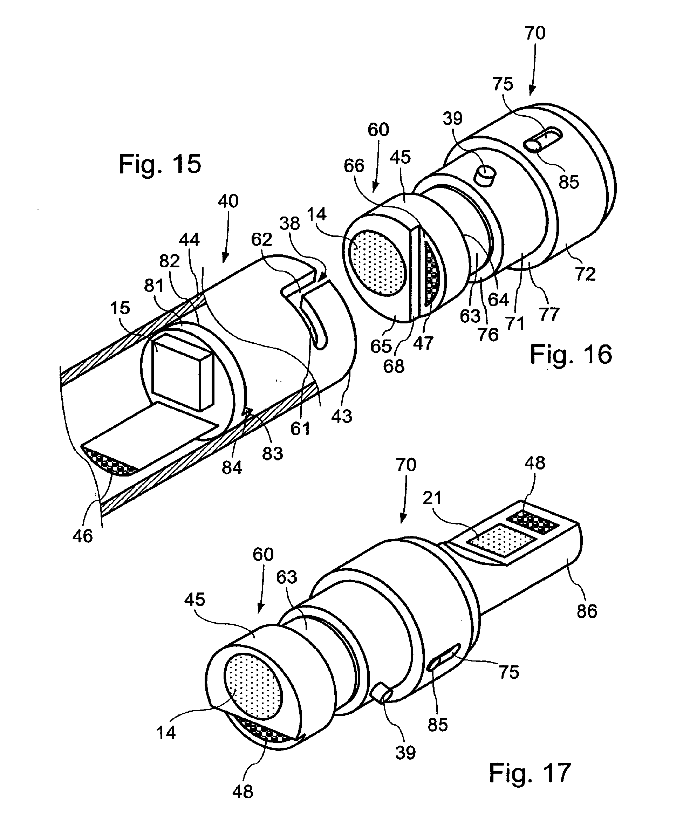

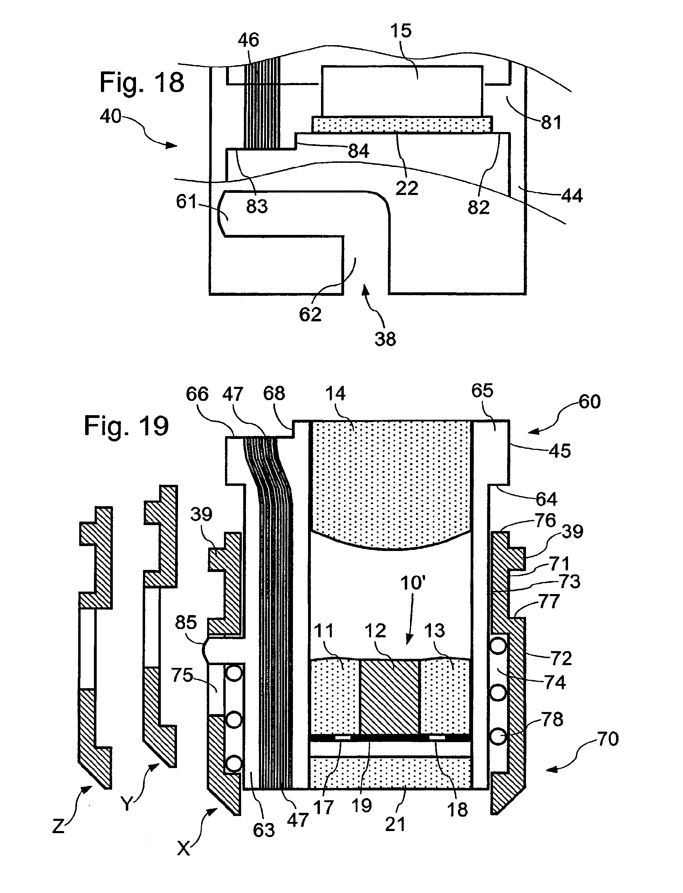

Les figures 15 et 18 montrent plus en détail la structure de l'embout distal 40 d'une sonde vidéoendoscopique. Cet embout comprend une cloison transversale 81 fixé à l'intérieur du tube cylindrique 44 et dont la face distale 82 forme une saillie mâle excentrée 84 présentant par exemple un profil externe en forme d'arc de cercle dont la partir courbe est confondue avec la face interne du tube 44. La cloison 81 dispose d'un premier orifice longitudinal débouchant sur la face distale 82 de la cloison et dont la partie proximale loge le capteur CCD 15, et d'un second orifice longitudinal excentré, débouchant sur la face distale 83 de la saillie 84, logeant l'extrémité distale du faisceau de fibres d'éclairage 46 de la sonde vidéoendoscopique. L'extrémité distale du tube 44 comporte deux fentes 38 en forme de baïonnettes, diamétralement opposées, présentant chacune une portion distale 62 ouverte, s'étendant longitudinalement et une portion proximale transversale fermée 61.

Les figures 16 et 19 montrent plus en détail la structure d'une tête amovible à visée axiale, verrouillable sur l'embout distal 40 de sonde vidéoendoscopique représentée sur la figure 15. Cette tête comprend une pièce proximale 60 de forme cylindrique, logeant les composants optiques et la fibre d'éclairage, intégrés dans la tête, et dont la partie distale est ceinturée de façon coulissante par une pièce de verrouillage 70 de forme tubulaire cylindrique. La pièce proximale 60 présente une partie proximale 45 dont le diamètre externe est compatible avec le diamètre interne du tube 44 de l'embout distal 40, et une partie distale 63 présentant un diamètre plus faible et comportant un doigt radial externe 85. La face proximale 65 de la pièce proximale 60 dispose d'un renfoncement femelle 68 destiné à loger lors du verrouillage la saillie mâle 62 située au niveau de la face distale 58 de la cloison transversale 57 de l'embout distal 40. Ce renfoncement 68 présente par exemple un profil externe en forme d'arc de cercle. La pièce proximale 60 comprend en outre un premier orifice longitudinal débouchant sur sa face proximale 65 et logeant un dispositif optique comportant notamment une lentille proximale 14, et un second orifice longitudinal excentré débouchant sur la face proximale 66 du renfoncement 68 et logeant le faisceau de fibres d'éclairage 47 de la tête amovible.

La pièce de verrouillage 70 dont le diamètre de l'orifice longitudinal interne est compatible avec le diamètre externe de la partie distale 63 de la pièce proximale 60, comporte une partie proximale 71 présentant un diamètre externe sensiblement identique au diamètre externe de la partie proximale 45 de la pièce 60 et disposant de deux doigts 39 radiaux externes, diamétralement opposés, et une partie distale 72 d'un diamètre externe identique au diamètre externe du tube 44 de l'embout distal 40 et disposant d'une fente longitudinale 75 dans laquelle est destiné à coulisser le doigt latéral externe 85 de la pièce proximale 60.

Un ressort hélicoïdal 78 ceinturant l'extrémité distale de la partie distale 63 de la pièce 60, est logé dans le volume annulaire ménagé à cet effet dans la partie distale 72 de la pièce de verrouillage 70. Ce ressort est prévu pour exercer une pression longitudinale sur le doigt latéral 85 solidaire de la partie distale 63 de la pièce 60, cette pression tendant à repousser le doigt 85 vers l'extrémité proximale de la fente 75.

Dans ces conditions, l'adaptation de la tête distale amovible sur l'extrémité distale 40 de la sonde vidéoendoscopique peut être effectuée suivant les trois phases successives suivantes.

Durant une phase X d'introduction correspondant à la configuration illustrée par la figure 16, l'extrémité proximale 45 de la pièce 60 est introduite dans l'extrémité distale du tube 44 de la sonde, et ce jusqu'à ce que les deux doigts radiaux externes 39 solidaires de la pièce de verrouillage 70 soient engagés dans les parties distales longitudinales 62 des fentes 38.

Durant une phase suivante Y de compression, l'opérateur exerce une force longitudinale sur l'extrémité proximale 45 de la tête amovible dans le tube 44. Dans cette configuration, la face proximale 65 de la pièce 60 est en contact avec la face distale 83 de la saillie 84 logeant le faisceau de fibres d'éclairage 46 de la sonde. Les doigts radiaux 39 se trouvent au niveau des extrémités proximales des parties longitudinales 62 des fentes 38. L'extrémité proximale 71 de la pièce de verrouillage 70 est complètement engagée dans l'extrémité distale du tube 44, de manière à ce que sa face proximale 75 soit en contact avec la face distale 64 de la partie proximale cylindrique de la pièce 60, et que la face proximale 77 du tube distal 72 de la pièce 70 soit en contact avec la face distale 43 du tube 44. Le ressort 78 est comprimé au maximum de telle manière que le doigt radial 85 solidaire de la partie distale 63 de la pièce 60 soit positionné dans l'extrémité distale de la fente 75.

Durant une phase Z de verrouillage, l'opérateur fait tourner la tête amovible dans le tube distal 44 de la sonde dans le sens contraire des aiguilles d'une montre pour positionner les doigts 39 au niveau des extrémités fermées des parties transversales 62 des fentes 38. Au terme de cette phase, la face proximale 65 de la partie proximale 45 de la pièce 60 est en contact avec la face distale 82 de la cloison transversale 81, de telle manière que les axes optiques du capteur CCD 15 et du système optique logé dans la tête amovible soient confondus. La saillie 84 de la cloison transversale 81 se trouve logée dans le renfoncement 68 ménagé dans la face proximale 65 de la partie distale 45 de la pièce 60, de telle façon que la face distale 83 de la saillie 84 soit en contact avec la face proximale 66 du renfoncement 68, et que l'extrémité distale du faisceau de fibres d'éclairage 46 de la sonde soit en contact étroit avec l'extrémité proximale du faisceau de fibres d'éclairage 47 de la tête. L'extrémité proximale 71 de la pièce 70 reste complètement engagée dans l'extrémité distale du tube 44 de manière à ce que la face proximale 77 du tube distal 725 reste en contact avec la face distale 43 du tube 44. Le ressort 78 se trouve légèrement détendu par rapport à la phase Y de compression, le doigt radial 85 solidaire de la pièce 60 étant positionné en partie médiane de la fente 75.

Le principe de verrouillage décrit ci-avant présente un haut niveau de sécurité en interdisant toute possibilité de déverrouillage accidentel. En effet, le déverrouillage de la tête amovible exige que l'opérateur repousse à l'aide d'un outil pointu le doigt 85 vers l'extrémité distale de la fente 75, puis de faire tourner la tête amovible dans le sens des aiguilles d'une montre, de manière à permettre de dégager les doigts 39 des fentes 38.

Il est à noter que le système mécanique constitué par l'emboîtement de la saillie 84 dans le renfoncement 68 constitue avantageusement un piège à lumière qui évite toute pollution du capteur CCD 15 par des rayons lumineux parasites.

La figure 17 représente une tête distale amovible à visée latérale, comportant le même dispositif de verrouillage que la tête à visée axiale représentée sur les figures 16 et 19.

Par rapport à la tête à visée axiale, la tête à visée latérale comporte en outre une extension cylindrique 86 intégrant un prisme déviateur distal dont la face d'entrée se trouve située derrière la fenêtre d'observation 21, et la courbure à 90° du faisceau de fibres d'éclairage dont l'extrémité distale se trouve logée dans une fenêtre d'illumination latérale 48.

Pour des raisons de sécurité de verrouillage, le tube 72 formant la partie distale de la pièce 70 peut être avantageusement allongé de manière à coiffer complètement l'extrémité 86 de la partie distale 63 de la pièce 60 et convenablement échancré de manière à ne pas obturer les fenêtres latérales d'observation 21 et d'illumination 48. Dans ces conditions, l'extrémité distale du tube 72 peut être obturée par une cloison transversale afin d'y intégrer un ressort de verrouillage (en remplacement du ressort 78) entre la face proximale de cette cloison et la face distale de la pièce 86.

## Revendications

1. Objectif distal de vidéoendoscope pour former sur la surface sensible d'un capteur vidéo (15) logé dans l'embout distal (40) d'un vidéoendoscope une unique image composite (34) formée de deux images (35, 36) d'une cible observée, vue sous deux angles différents,
   **caractérisé en ce qu'**il comprend un unique composant optique de dédoublement d'image (10, 10') et une lentille convergente proximale (14) disposée en position proximale par rapport au composant optique de dédoublement (10, 10'), le composant optique de dédoublement d'image étant constitué de deux portions (3, 4 ; 11, 13) de lentille convergente (1, 2) identiques, traversées chacune par l'axe optique (6, 7) de la lentille convergente (1, 2) d'où elles sont issues, et d'un élément central opaque (12) maintenant les deux portions de lentilles espacées l'une de l'autre, de manière à ce que les axes optiques des deux portions de lentilles soient espacés l'un de l'autre, la lentille convergente proximale coopérant avec chacune des deux portions de lentilles pour former l'image composite (34).

2. Objectif selon la revendication 1,
   **caractérisé en ce que** le composant optique de dédoublement d'image (10, 10') est associé à un masque opaque (19) présentant deux orifices (17, 18) formant deux diaphragmes, centrés respectivement sur les axes optiques des deux portions de lentilles (3, 4 ; 11, 13)

3. Objectif selon la revendication 1 ou 2,
   **caractérisé en ce que** les dimensions de l'élément central opaque (12) sont déterminées de manière à éviter un chevauchement de champs images (35, 36) produits par les portions de lentilles (11, 13) au travers de la lentille convergente proximale (14).

4. Objectif selon l'une des revendications 1 à 3,
   **caractérisé en ce que** le composant optique de dédoublement d'image (10') est obtenu par une découpe suivant un cylindre (8) de section droite circulaire, centré sur l'élément opaque (12) et dont l'axe longitudinal est parallèle aux axes optiques des portions de lentilles (3, 4).

5. Objectif selon la revendication 4,
**caractérisé en ce qu'**il comprend en outre une lentille divergente (23) disposée en position distale par rapport au composant optique de dédoublement (10, 10').

6. Objectif selon l'une des revendications 1 à 4,
**caractérisé en ce qu'**il comprend en outre des moyens de réflexion (20) disposés en position distale, pour introduire une déviation de l'angle de visée du dispositif de dédoublement d'image.

7. Objectif selon la revendication 6,
**caractérisé en ce qu'**il comprend en outre une lentille divergente (23) disposée en position distale par rapport aux moyens de réflexion (20).

8. Objectif selon l'une des revendications 1 à 7,
**caractérisé en ce qu'**il se présente sous la forme d'une tête optique (41, 42, 41', 42') comprenant des moyens de fixation pour se fixer de manière amovible sur l'embout distal (40) d'un vidéoendoscope, pour former une image dédoublée sur la surface sensible d'un capteur vidéo (15) logé dans l'embout distal (40).

9. Objectif selon la revendication 8,
**caractérisé en ce que** les moyens de fixation sont du type doigt (39) destiné à coopérer avec une fente en forme de baïonnette (38) et comprennent des moyens d'accouplement pour assurer un alignement correct de l'axe optique de la tête optique avec le capteur vidéo.

10. Objectif selon la revendication 9,
**caractérisé en ce qu'**il comprend un faisceau de fibres d'éclairage (47, 48) dont l'extrémité distale est destinée à éclairer la cible (46) et dont l'extrémité proximale est destinée à être couplée à l'extrémité distale d'un faisceau de fibres d'éclairage équipant le vidéoendoscope, les moyens d'accouplement assurant un alignement correct du faisceau de fibres d'éclairage avec celui du vidéoendoscope.

11. Objectif selon l'une des revendications 8 à 10,
**caractérisé en ce que** les moyens de fixation sont formés sur un tube de verrouillage (70) mobile axialement sur la tête optique entre des positions distale et proximale, et ramené vers la position distale par des moyens de rappel élastiques (78), les moyens d'accouplement (68, 84) étant de type mâle-femelle et étant disposés de manière à arriver en position accouplée en fin de course de verrouillage des moyens de verrouillage (38, 39), sous l'effet des moyens de rappel élastiques.

12. Vidéoendoscope comprenant un embout distal logeant un capteur vidéo (15),
**caractérisé en ce que** le capteur vidéo (15) est couplé à un objectif selon l'une des revendications 8 à 11.

13. Vidéoendoscope selon la revendication 12,
**caractérisé en ce qu'**il comprend des moyens de calcul de la distance d'observation d'un point (A) de la cible à partir de la distance sur un écran de visualisation sur lequel est affichée l'image composite de la cible formée sur le capteur vidéo (15) entre deux points images (A3, A4) du point (A) de la cible, générés par le dispositif de dédoublement d'image.

14. Vidéoendoscope selon la revendication 12 ou 13,
**caractérisé en ce qu'**il comprend des moyens de calcul de la distance entre un premier et un second points (A, B) de la cible à partir des positions respectives sur un écran de visualisation sur lequel est affichée l'image composite de la cible formée sur le capteur vidéo (15) de deux points images (A3, A4) du premier point (A) de la cible et de deux points images (B3, B4) du second point (B), générés par le dispositif de dédoublement d'image.

15. Vidéoendoscope selon l'une des revendications 12 à 14,
**caractérisé en ce qu'**il comprend des moyens pour appliquer à une portion de l'image composite représentant la cible observée, un traitement d'agrandissement, de manière à afficher une seule des deux images de la cible, constituant l'image composite.

**16.** Vidéoendoscope selon l'une des revendications 12 à 15,
**caractérisé en ce qu'**il comprend une paire de lunettes (55) de vision stéréo comprenant deux mini écrans vidéo (56, 57) vers lesquels sont envoyées respectivement les deux images (51, 52) dédoublées de la cible, après avoir subi un traitement d'agrandissement.

**17.** Vidéoendoscope selon l'une des revendications 12 à 16,
**caractérisé en ce que** le capteur vidéo (15) est un capteur CCD.

**Claims**

**1.** A videoendoscope distal objective to form on the sensitive surface of a video sensor (15) housed in the distal end part (40) of a videoendoscope a single composite image (34) made up of two images (35, 36) of an observed target, viewed from two different angles,
**characterized in that** it comprises a single image splitting optical component (10, 10') and a proximal converging lens (14) disposed in proximal position in relation to the splitting optical component (10, 10'), the image splitting optical component being made up of two identical portions (3, 4; 11, 13) of a converging lens (1, 2), each passed through by the optical axis (6, 7) of the converging lens (1, 2) from which they come, and of a central opaque element (12) maintaining the two portions of lenses spaced apart, so that the optical axes of the two portions of lenses are spaced apart, the proximal converging lens cooperating with each of the two portions of lenses to form the composite image (34).

**2.** Objective according to claim 1,
**characterized in that** the image splitting optical component (10, 10') is associated with an opaque mask (19) having two orifices (17, 18) forming two diaphragms, respectively centered on the optical axes of the two portions of lenses (3, 4; 11, 13).

**3.** Objective according to claim 1 or 2,
**characterized in that** the dimensions of the central opaque element (12) are determined so as to avoid any overlapping of image fields (35, 36) produced by the portions of lenses (11, 13) through the proximal converging lens (14).

**4.** Objective according to one of claims 1 to 3,
**characterized in that** the image splitting optical component (10') is obtained by cutting along a cylinder (8) with a circular straight section, centred on the opaque element (12) and the longitudinal axis of which is parallel to the optical axes 35 of the portions of lenses (3,4).

**5.** Objective according to claim 4,
**characterized in that** it further comprises a diverging lens (23) disposed in distal position in relation to the splitting optical component (10, 10').

**6.** Objective according to one of claims 1 to 4,
**characterized in that** it further comprises reflection means (20) disposed in distal position, to introduce a deviation of the viewing angle of the image splitting device.

**7.** Objective according to claim 6,
**characterized in that** it further comprises a diverging lens (23) disposed in distal position in relation to the reflection means (20).

**8.** Objective according to one of claims 1 to 7,
**characterized in that** it is in the shape of an optical head (41, 42, 41', 42') comprising fixing means so as to be fixed in a removable manner onto the distal end part (40) of a videoendoscope, to form a split image on the sensitive surface of a video sensor (15) housed in the distal end part (40).

**9.** Objective according to claim 8,
**characterized in that** the fixing means are of the finger type (39) intended to cooperate with a bayonet-shaped slit (38) and comprise coupling means to ensure a correct alignment of the optical axis of the optical head with the video sensor.

10. Objective according to claim 9,
**characterized in that** it comprises a bundle of lighting fibres (47, 48) the distal end of which is intended to light up the target (46) and the proximal end of which is intended to be coupled to the distal end of a bundle of lighting fibres equipping the videoendoscope, the coupling means ensuring a correct alignment of the bundle of lighting fibres with that of the videoendoscope.

11. Objective according to one of claims 8 to 10,
**characterized in that** the fixing means are formed on a locking tube (70) axially mobile on the optical head between distal and proximal positions, and brought back towards the distal position by elastic return means (78), the coupling means (68, 84) being of male-female type and being disposed so as to reach a coupled position at the end of the locking travel of the locking means (38, 39), under the effect of the elastic return means.

12. A videoendoscope comprising a distal end part housing a video sensor (15), **characterized in that** the video sensor (15) is coupled to an objective according to one of claims 8 to 11.

13. Videoendoscope according to claim 12,
**characterized in that** it comprises means for calculating the viewing distance of a point (A) of the target from the distance on a display unit displaying the composite image of the target formed on the video sensor (15) between two image points (A3, A4) of the point (A) of the target, generated by the image splitting device.

14. Videoendoscope according to claim 12 or 13,
**characterized in that** it comprises means for calculating the distance between a first and a second point (A, B) of the target from the respective positions on a display unit displaying the composite image of the target formed on the video sensor (15) of two image points (A3, A4) of the first point (A) of the target and of two image points (B3, B4) of the second point (B), generated by the image splitting device.

15. Videoendoscope according to one of claims 12 to 14,
**characterized in that** it comprises means for applying to a portion of the composite image representing the observed target, an enlargement process, so as to display only one of the two images of the target, constituting the composite image.

16. Videoendoscope according to one of claims 12 to 15,
**characterized in that** it comprises a pair of stereo vision glasses (55) comprising two mini video screens (56, 57) to which the two split images (51, 52) of the target are respectively sent, after an enlargement process.

17. Videoendoscope according to one of claims 12 to 16,
**characterized in that** the video sensor (15) is a CCD sensor.

**Patentansprüche**

1. Distales Objektiv für Videoendoskop zur Bildung eines einzigen, aus zwei Bildern (35, 36) eines beobachteten Zieles aus zwei verschiedenen Blickwinkeln zusammengesetzten Bildes (34) auf der empfindlichen Oberfläche eines Bildsensors (15), der im distalen Ende (40) eines Videoendoskops angeordnet ist, **dadurch gekennzeichnet, dass** es ein einziges optisches Bauteil zur Bildverdopplung (10, 10') umfasst und eine proximale Sammellinse (14), die relativ zum optischen Bauteil zur Bildverdopplung (10, 10') in proximaler Stellung angeordnet ist, wobei das optische Bauteil zur Bildverdopplung aus zwei identischen Teilen (3, 4; 11, 13) einer Sammellinse (1, 2) besteht, die jeweils von der optischen Achse (6, 7) der Sammellinse (1, 2) durchquert werden, aus der sie hervorgegangen sind, und aus einem zentralen lichtundurchlässigen Teil (12), das die beiden Linsenteile voneinander beabstandet hält, derart, dass die optischen Achsen der beiden Linsenteile voneinander beabstandet sind, wobei die proximale Sammellinse mit jedem der beiden Linsenteile zusammenarbeitet, um das zusammengesetzte Bild (34) zu bilden.

2. Objektiv nach Patentanspruch 1, **dadurch gekennzeichnet, dass** dem optischen Bauteil zur Bildverdopplung (10, 10') eine lichtundurchlässige Maske (19) zugeordnet ist, die zwei Öffnungen (17, 18) aufweist, die zwei Blenden bilden, die jeweils um die optischen Achsen der beiden Linsenteile (3, 4; 11, 13) zentriert sind.

3. Objektiv nach Patentanspruch 1 oder 2, **dadurch gekennzeichnet, dass** die Abmessungen des zentralen undurchsichtigen Teiles (12) derart festgelegt sind, dass ein Überlappen der Bildfelder (35, 36), die von den Linsenteilen (11, 13) durch die proximale Sammellinse (14) erzeugt werden, vermieden wird.

**4.** Objektiv nach einem der Patentansprüche 1 bis 3, **dadurch gekennzeichnet, dass** das optische Bauteil zur Bildverdopplung (10') durch ein Ausschneiden längs eines Zylinders (8) mit kreisförmigem Querschnitt erhalten wird, der um das undurchsichtige Teil (12) zentriert ist und dessen Längsachse zu den optischen Achsen der Linsenteile (3, 4) parallel ist.

**5.** Objektiv nach Patentanspruch 4, **dadurch gekennzeichnet, dass** es außerdem eine Zerstreuungslinse (23) umfasst, die relativ zum optischen Bauteil zur Verdopplung (10, 10') distal angeordnet ist.

**6.** Objektiv nach einem der Patentansprüche 1 bis 4, **dadurch gekennzeichnet, dass** es außerdem reflektierende Mittel (20) umfasst, die distal angeordnet sind, um eine Änderung des Sichtwinkels der Bildverdopplungsvorrichtung zu erreichen.

**7.** Objektiv nach Patentanspruch 6, **dadurch gekennzeichnet, dass** es außerdem eine Zerstreuungslinse (23) umfasst, die relativ zum reflektierenden Mittel (20) distal angeordnet ist.

**8.** Objektiv nach einem der Patentansprüche 1 bis 7, **dadurch gekennzeichnet, dass** es die Form eines optischen Kopfes (41, 42, 41', 42') hat, der Befestigungsmittel aufweist, um abnehmbar am distalen Endstück (40) eines Videoendoskops befestigt werden zu können, um auf der empfindlichen Oberfläche eines Bildsensors (15), der im distalen Endstück (40) aufgenommen wird, ein verdoppeltes Bild zu schaffen.

**9.** Objektiv nach Patentanspruch 8, **dadurch gekennzeichnet, dass** die Befestigungsmittel von der Art eines Stiftes (39) sind, der dazu bestimmt ist, mit einem Bajonett-förmigen Schlitz (38) zusammenzuarbeiten, und umfassen Kupplungsmittel, um eine korrekte Ausrichtung der optischen Achse des optischen Kopfes mit dem Bildsensor sicherzustellen.

**10.** Objektiv nach Patentanspruch 9, **dadurch gekennzeichnet, dass** es ein Beleuchtungsfaserbündel (47, 48) umfasst, dessen distales Ende dazu bestimmt ist, das Ziel (46) zu beleuchten, und dessen proximales Ende dazu bestimmt ist, an das distale Ende eines Beleuchtungsfaserbündels angekuppelt zu werden, mit dem das Videoendoskop ausgestattet ist, wobei die Kupplungsmittel eine korrekte Ausrichtung des Beleuchtungsfaserbündels mit dem des Videoendoskops sicherstellen.

**11.** Objektiv nach einem der Patentansprüche 8 bis 10, **dadurch gekennzeichnet, dass** die Befestigungsmittel auf einem Verriegelungsrohr (70) ausgebildet sind, das am optischen Kopf in Achsenrichtung zwischen distaler und proximaler Stellung beweglich ist und durch elastische Rückstellmittel (78) in die distale Stellung zurückgebracht wird, wobei die Kupplungsmittel (68, 84) vom Nut-Feder-Typ sind und derart angeordnet sind, dass sie unter der Wirkung der elastischen Rückstellmittel am Ende der Verriegelungsbewegung der Verriegelungsmittel (38, 39) die eingekuppelte Stellung erreichen.

**12.** Videoendoskop, ein distales Endstück umfassend, das einen Bildsensor (15) aufnimmt, **dadurch gekennzeichnet, dass** der Bildsensor (15) an ein Objektiv nach einem der Patentansprüche 8 bis 11 gekoppelt ist.

**13.** Videoendoskop nach Patentanspruch 12, **dadurch gekennzeichnet, dass** es Mittel zur Berechnung des Beobachtungsabstandes eines Punktes (A) des Zieles aus dem Abstand zwischen zwei durch die Bildverdopplungsvorrichtung erzeugten Bildpunkten (A3, A4) des Punktes (A) des Zieles auf einem Darstellungsbildschirm, auf dem das zusammengesetzte Bild des Zieles angezeigt wird, das auf dem Bildsensor (15) gebildet wird, umfasst.

**14.** Videoendoskop nach Patentanspruch 12 oder 13, **dadurch gekennzeichnet, dass** es Mittel zur Berechnung des Abstandes zwischen einem ersten und einem zweiten Punkt (A, B) des Zieles aus den jeweiligen Stellen zweier durch die Bildverdopplungsvorrichtung erzeugter Bildpunkte (A3, A4) des ersten Punktes (A) des Zieles und zweier durch die Bildverdopplungsvorrichtung erzeugter Bildpunkte (B3, B4) des zweiten Punktes (B) auf einem Darstellungsbildschirm, auf dem das zusammengesetzte Bild des Zieles angezeigt wird, das auf dem Bildsensor (15) gebildet wird, umfasst.

**15.** Videoendoskop nach einem der Patentansprüche 12 bis 14, **dadurch gekennzeichnet, dass** es Mittel umfasst, um auf einen Teil des zusammengesetzten Bildes, das das beobachtete Ziel darstellt, eine Vergrößerungsverarbeitung anzuwenden, um ein einziges der beiden Bilder des Zieles anzuzeigen, die das zusammengesetzte Bild

**15**

bilden.

16. Videoendoskop nach einem der Patentansprüche 12 bis 15, **dadurch gekennzeichnet, dass** es eine Stereosichtbrille (55) umfasst, die zwei Kleinbildschirme (56, 57) aufweist, denen jeweils eins der beiden verdoppelten Bilder (51, 52) des Ziels zugesandt werden, nachdem sie einer Vergrößerungsverarbeitung unterworfen wurden.

17. Videoendoskop nach einem der Patentansprüche 12 bis 16, **dadurch gekennzeichnet, dass** der Bildsensor (15) ein CCD-Sensor ist.

Fig. 1

Fig. 2a

Fig. 2b

Fig. 3a

Fig. 3b

Fig. 4

Fig. 5

Fig. 6

14  10,10'

A

B

30

15

50

52  B3  B4  51

A3  A4

Fig. 7

55

57  56

53

B4  A4

Fig. 9

Fig. 8

Fig. 10

Fig. 14

Fig. 12

Fig. 11

Fig. 13

Fig. 15

Fig. 16

Fig. 17

Fig. 18

40

46 15

81

84

22 44

61 83

82

62

38

Fig. 19

66 47 68 14 65 60

45

64

39 76 39

71

73

77

85 10'

72

75 11 12 13 74

78 70

Z Y 17 19 18

X 63 47 21

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4702229 A
- US 4820043 A
- US 6333812 B
- FR 2832516
- JP 11045349 B
- US 6100972 A
- WO 9620389 A
- US 4558691 A
- US 6476979 B
- US 4207594 A
- GB 2269453 A
- IL 156074
- DE 2847561
- US 4660982 A
- US 4980763 A
- US 5070401 A

- FR 2630538
- DE 3629435
- US 4873572 A
- US 20020137986 A
- US 6063023 A
- US 6361491 B
- US 5222477 A
- US 3932699 A
- FR 2619664
- FR 2704951
- FR 2705006
- US 4993830 A
- DE 3432583
- US 6411327 B
- US 20020089583 A
- US 4727859 A